# EUROPÄISCHE PATENTSCHRIFT

(11) **EP 1 690 503 B1**
(45) Veröffentlichungstag und Bekanntmachung des Hinweises auf die Patenterteilung: **24.07.2013**
(21) Anmeldenummer: 05003148.3
(22) Anmeldetag: 15.02.2005
(51) Int. Cl.: A61B 17/15, A61B 19/00

(54) **Benutzerführung bei der Justierung von Knochenschneidblöcken**
User guidance for adjusting the cutting guides for the bones
Mode d'utilisation pour le réglage de la position des guides de coupe d'un os

(43) Veröffentlichungstag der Anmeldung: 16.08.2006
(73) Patentinhaber: BrainLAB AG, 85622 Feldkirchen (DE)
(72) Erfinder: Neubauer, Timo, 85622 Feldkirchen (DE); Plassky, Norman, 99099 Erfurt (DE); Brack, Christian, Dr., 86420 Diedorf (DE); Götte, Hubert, 80333 München (DE)
(74) Vertreter: Schwabe - Sandmair - Marx

(56) Entgegenhaltungen:
- EP-A- 1 430 842
- WO-A-2004/019792
- DE-U1- 20 202 615
- DE-U1- 20 303 643
- US-A1- 2002 068 942
- US-A1- 2002 198 531

## Beschreibung

Die Erfindung betrifft die Benutzerführung bei der Justierung von Knochenschneidblöcken, und insbesondere betrifft sie einen speziell hierfür gestalteten Schneidblock.

Beispielsweise bei der computerunterstützten Knie-Arthroplastie wird gewöhnlich ein Schneidblock verwendet, der eine Schneidführung aufweist, an welcher eine mitunter räumlich verstellbare z.B. drehbare Ortungsreferenz angebracht ist, um eine bestmögliche Sichtbarkeit der Ortungsreferenz für verschiedene Setups zu erlauben. Mittels der Ortungsreferenz und eines medizintechnischen Navigationssystems wird versucht, den Schneidblock so zu positionieren, dass die Schneidführung in der geplanten Schnittebene zu liegen kommt.

Aus der WO 2004/017843 A1 ist ein Führungsblock zur Verwendung bei chirurgischen Eingriffen bekannt, der ein Fixierungsteil aufweist, das an einem Knochen befestigt ist, sowie ein Führungsteil, dass relativ zu dem Fixierungsteil beweglich und in der Position justierbar ist. Leuchtdioden werden verwendet, um die Position des Führungsteils anzuzeigen. Die DE 102 07 035 A1 offenbart eine Schablone zur Führung eines chirurgischen Bearbeitungswerkzeuges. Diese weist eine Befestigungseinrichtung zur Festlegung der Schablone an einem zu bearbeitenden Knochen mittels drei Knochenschrauben auf.

Aus der DE 202 02 615 U1 ist eine Schablone zur Führung eines chirurgischen Bearbeitungswerkzeuges mit einer Befestigungseinrichtung zur Festlegung der Schablone an einem zu bearbeitenden Knochen bekannt, wobei ein Referenzelement für die Navigation mit einem Navigationssystem fest an einer Halterung der Schablone angeordnet ist.

Aus der EP 1 430 842 A1 ist ein medizinisches Instrument bekannt, welches eine verstellbare Schablone und fest am Instrument angebrachte Referenzmarken aufweist, wobei das Instrument an einem Knochen verschraubt wird.

Die Verwendung einer Justierungseinrichtung zur Einstellung der Schnittebene in den knochentypischen Freiheitsgraden Slope, Varus-Valgus und Resektionstiefe nach den Vorgaben des Navigationssystems (Soll-Schnittebene) die Einstellung der Schnittebene, gestaltet sich insbesondere dann schwierig, wenn der Knochenschneidblock (insbesondere die Justierungseinrichtung) so am Knochen befestigt ist, dass die Bedienelemente nicht eindeutig den einzustellenden Freiheitsgraden der Schnittebene zugeordnet sind, so dass die Bedienung eines Elementes mitunter die Veränderung in mehr als einem Freiheitsgrad zur Folge hat oder zwei Bedienelemente betätigt werden müssen, um die Verstellung in einem Freiheitsgrad zu erreichen. Es hängt dann sehr von dem Können und der Erfahrung des Bedieners ab, den günstigsten Weg zu finden, um in kürzester Zeit den Justiervorgang abzuschließen. Das heißt, obwohl durch die Navigation die Soll-Schnittebene vorgegeben ist, ist es oft schwierig, ohne Unterstützung die Schneidführung nach diesen Vorgaben einzustellen, vor allem wenn der Schneidblock ungünstig am Knochen angeordnet ist, was mitunter ein iteratives Vorgehen erforderlich macht, da eine gerichtete Justierung durch Abhängigkeit der Funktionsglieder nicht möglich ist.

Es ist die Aufgabe der vorliegenden Erfindung, die Benutzerführung bei der Justierung von Knochenschneidblöcken zu vereinfachen, so dass die Schneidführung schnell und genau in die Soll-Schnittebene gebracht werden kann.

Diese Aufgabe wird erfindungsgemäß durch einen Knochenschneidblock gemäß dem Anspruch 1 gelöst. Die Unteransprüche definieren bevorzugte Ausführungsformen der Erfindung.

Was den Knochenschneidblock betrifft, so beruhen die erfindungsgemäßen Vorteile insbesondere darauf, dass ein Registrierungselement bereitgestellt wird, mit dem die räumliche Lage der Justierungseinrichtung bestimmbar ist. Das Registrierungselement sorgt also mit anderen Worten dafür, dass die Position der Justierungseinrichtung am Knochen ermittelt werden kann, was mitunter auch Informationen über die Lage der Ortungsreferenz voraussetzt. Weiß man über Position und Orientierung der Justierungseinrichtung am Knochen bescheid, kann man voraussagen, welche Wirkungen die Einstellungen an der Justierungseinrichtung auf die Schnittebene der Schneidführung haben werden. Mit diesem Wissen ist es möglich, den Benutzer zu unterstützen, so dass er die Schneidführung schnell und genau einstellen kann. Der einstellbare Schneidblock mit diesen Registrierungsmerkmalen und der Justierungseinrichtung gestattet insbesondere in Kombination mit einem Softwarealgorithmus und einer Benutzerschnittstelle die exakte Bestimmung der notwendigen Einstellbewegungen sowie die Führung des Benutzers bei der Erzeugung dieser Einstellbewegungen mithilfe der Justierungseinrichtung. Dies kann für jedwede Anordnung des Schneidblocks am Knochen durchgeführt werden (anterior-posterior, medial, lateral,...), da die Position der Justierungseinrichtung im Bezug auf die Knochenachse und -ebenen ermittelt wird, die Struktur des Knochenschneidblockes (insbesondere der Justierungseinrichtung) bekannt ist (z.B. im Navigationssystem hinterlegt), und deshalb errechnet werden kann, wie die Justierungseinrichtung betätigt werden muss, um die gewünschten Werte für die Schnittebene einzustellen.

Mit anderen Worten: Bisher wurde zwar die Soll-Schnittebene per Bildschirmanzeige angegeben, jedoch nicht der Weg dorthin. Bei Standard-Anterior-Anordnungen ist es auch nicht allzu schwierig, genau ein Einstellmittel einem gewünschten Freiheitsgrad zuzuordnen. Damit aber ein einstellbarer Knochenschneidblock in allen möglichen Anordnungen (Befestigung um das gesamte Bein herum) in der Praxis gut umsetzbar möglich wird, ist es notwendig, den Benutzer erfindungsgemäß zu führen, während er die Justierungseinrichtung bedient, da er bei ungünstigen Schneidblockanordnungen intuitiv nicht vorhersagen kann, welchen Effekt eine bestimmte Manipulation an der Justierungseinrichtung auf die resultierende Schnittebene haben wird.

Der Vorteil der Erfindung ist also, dass man die räumliche Lage der Justierungseinrichtung bestimmt, so dass man dem Benutzer die Ist-Schnittebene, die Soll-Schnittebene, den Unterschied zwischen den beiden und die Instruktionen übermitteln kann, wie er durch Manipulation der Justierungseinrichtung die Soll-Schnittebene einstellen kann. Diese Benutzerführung ist bei allen möglichen Anordnungen des Schneidbocks um den Knochen herum anwendbar. Sie erspart Zeit und gestattet die genaue Schnittebeneneinstellung.

Die Justierungseinrichtung kann Einstellmittel aufweisen, mittels denen die Schneidführung um zwei nichtparallele Achsen gedreht und in der Höhe verstellt werden kann, insbesondere jeweils ein Einstellmittel für jeden Freiheitsgrad, speziell drei Handrad-Schraubeinstellvorrichtungen.

Das Registrierungselement umfasst eine punktförmige Referenz oder eine Referenz mit einer zweidimensionalen Anschalung, wobei die Referenz fest an der Justierungseinrichtung angeordnet ist. Es ist möglich, die Referenz der Justierungseinrichtung an einem für den einzelnen Knochenschneidblock charakteristischen Ort anzuordnen. Außerdem besteht die Möglichkeit, die Referenz der Justierungseinrichtung in einem vorgegebenen Abstand von der Ortungsreferenz anzuordnen oder, für verschiedene Konfigurationen (verschiedene Anordnungen der Schneidführung zur Justierungseinrichtung), in einem definierten voreinstellbaren Abstand von der Ortungsreferenz.

Die Referenz kann eine Vertiefung an einer Stelle der Oberfläche der Justierungseinrichtung sein, also eine "eindimensionale" Punktreferenz. Eine solche Vertiefung kann im Registrierungsprozess beispielsweise mit einem navigierten Zeigestab (Pointer) angefahren und positionsmäßig bestimmt werden (Navigationssystem).

Die Referenz kann auch eine zweidimensionale Ausdehnung haben, insbesondere eine Bohrung an einer Stelle der Justierungseinrichtung sein oder zwei Vertiefungen an der Oberfläche der Justierungseinrichtung haben, wobei auch die räumliche Lage der zweidimensionalen Elemente im Registrierungsprozess durch einen navigierten Zeigestab erfassbar ist.

Bei einer anderen Ausführungsform der vorliegenden Erfindung wird die räumliche Lage der Justierungseinrichtung in Bezug auf die Ortungsreferenz vorbestimmt eingestellt. Eine vorhandene arretierbare Schnittstelle zwischen Justierungseinrichtung inklusive Registrierungselement und Schneidführung inklusive Ortungsreferenz erlaubt dabei nur definierte Relativstellungen beider Elemente, wobei die entsprechenden Informationen im Navigationssystem hinterlegt sind.

Gemäß einer weiteren Ausführungsform ist es vorstellbar, dass die Registrierungsvorrichtung eine Erfassungs- und Anzeigevorrichtung für die Winkel-Relativstellung zwischen der Ortungsreferenz und der Justierungseinrichtung umfasst.

Ein offenbartes, nicht zur Erfindung gehöriges Verfahren zur Benutzerführung bei der Einstellung der Schnittebene der Schneidführung eines Knochenschneidblocks mit einer Schneidführungs-Justierungseinrichtung umfasst die folgenden Schritte:
- eine Soll-Schnittebene wird festgelegt;
- mit Hilfe eines medizinischen Navigationssystems und einer Ortungsreferenz, die an der Schneidführung angebracht ist, wird eine Ist-Schnittebene räumlich bestimmt;
- mittels eines Registrierungselements wird die räumliche Lage der Justierungseinrichtung in Bezug auf den Knochen bestimmt; und
- in Abhängigkeit von der räumlichen Lage der Justierungseinrichtung werden Justierungsanweisungen ausgegeben, die den Benutzer, ausgehend von der Ist-Schnittebene, bei der Einstellung der Soll-Schnittebene unterstützen.

Ein solches Verfahren kann mit einem Knochenschneidblock durchgeführt werden, wie er oben im Einzelnen beschrieben wurde. Insbesondere ist es von Vorteil, wenn mittels einer Bildschirmausgabe, insbesondere der Bildschirmausgabe des Navigationssystems, die Justierungsanweisungen für einzelne Einstellmittel der Justierungseinrichtung ausgegeben werden. Eine solche Bildausgabe ist vorteilhaft, sie kann auch durch eine akustische Ausgabe unterstützt werden. Ebenfalls unterstützend wirkt sich eine farbliche oder anderweitige Markierung (z.B. Nummerierung) der Einstellmittel aus, da über eine entsprechende Bildschirmausgabe eine eindeutige Identifizierung der aktuell zu verwendenden Einstellmittel deren Auffinden am Schneidblock durch den Benutzer erleichtern kann.

Die Erfindung wird im Weiteren anhand zweier bevorzugter Ausführungsformen näher erläutert. Sie kann alle hierin genannten Merkmale einzeln oder in jedweder Kombination umfassen. In den beiliegenden Zeichnungen zeigen:
- Figur 1: eine Anordnung eines erfindungsgemäßen Schneidblocks am Unterschenkelknochen im navigierten Umfeld;
- Figur 2: eine vergrößerte Darstellung eines erfindungsgemäßen Schneidblocks mit einem eindimensionalen Registrierungselement;
- Figur 3 bis Figur 5: verschiedene Schneidführungs-Konfigurationen bzw. Anordnungen der Schneidführungen an einem Schneidblock;
- Figur 6: erfindungsgemäßen Schneidblock mit Registrierungsebene aufgespannt durch die Drehachse der Ortungsreferenz und den Registrierungspunkt (eindimensionales Registrierungselement) ;
- Figur 7: erfindungsgemäßen Schneidblock mit Registrierungsebene und Symmetrieebene der Justierungseinrichtung und deren Verhältnis zueinander;
- Figur 8: erfindungsgemäßen Schneidblock (mit dargestellter Symmetrieebene der Justierungseinrichtung) befestigt am Unterschenkel (mit dargestellter Sagittalebene);
- Figur 9: eine vergrößerte Darstellung eines erfindungsgemäßen Schneidblocks mit einem zweidimensionalen Registrierungselement;
- Figur 10: erfindungsgemäßen Schneidblock bei der Registrierung seiner räumlichen Lage mittels navigierbarem Zeigestab;
- Figur 11: erfindungsgemäßen Schneidblock mit Registrierungsebene deren Normalenvektor aus den beiden Registrierungspunkten gebildet wird und die gleichzeitig parallel zur Symmetrieebene des Schneidblocks ist;
- Figur 12: Mögliche Bildschirmausgabe zur Führung des Benutzers bei der Registrierung des Schneidblocks; und
- Figur 13 bis Figur 16: Mögliche Bildschirmausgabe zur Führung des Benutzers bei der Bedienung der Justierungseinrichtung in den verschiedenen Freiheitsgraden.

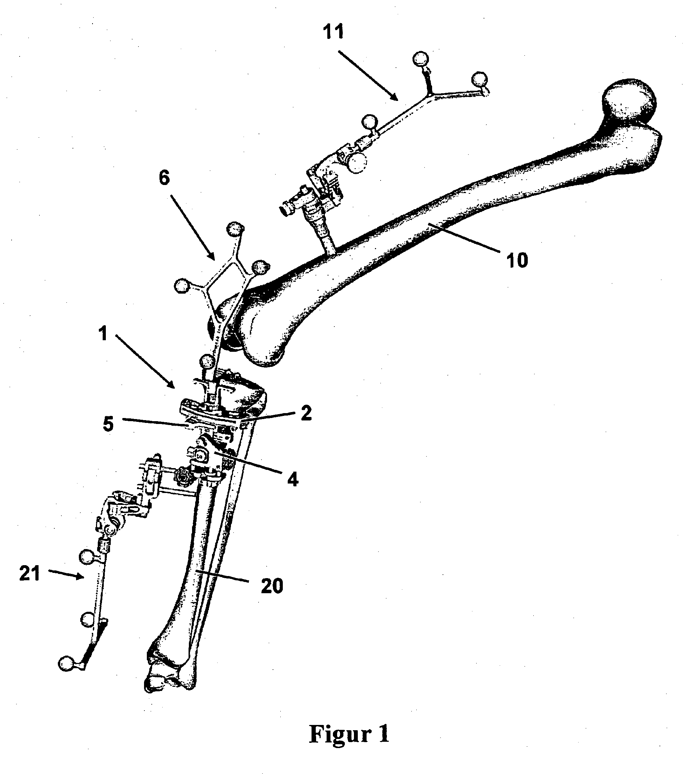

In der Figur 1 ist das Umfeld aufgezeigt, in dem ein erfindungsgemäßer Knochenschneidblock verwendet und ein erfindungsgemäßes Benutzerführungsverfahren durchgeführt wird.

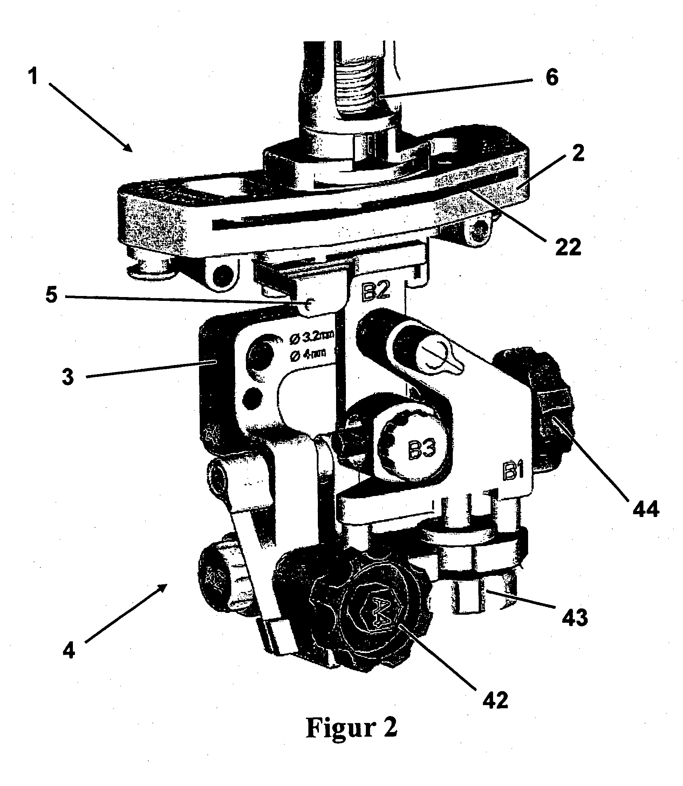

Der Knochenschneidblock trägt insgesamt das Bezugszeichen 1; seine Einzelkomponenten sind in Figur 2 deutlicher erkennbar. Der Knochenschneidblock 1 ist an einem Unterschenkelknochen 20 befestigt, und zwar mit seiner Befestigung 3 (Figur 2), insbesondere angeschraubt oder von Schanzschrauben, Kirschnerdrähten oder ähnlichen Befestigungsmitteln gehalten. An die Befestigung 3 anschließend befindet sich die Justierungseinrichtung 4, an welcher die Schneidführung 2 fixiert ist.

Oben auf der Schneidführung 2 ist drehbar die Ortungsreferenz 6 angeordnet, die eine Gruppe reflektierender Marker trägt.

In Figur 1 ist noch zu sehen, dass am Unterschenkelknochen 20 eine Referenz 21 und am Oberschenkelknochen 10 eine weitere Referenz 11 vorgesehen sind. Die Knochen und die Schneidführung 2 werden dadurch geortet und navigiert, und zwar durch ein hier nicht dargestelltes Navigationssystem, das zwei Trackingkameras, eine Infrarotlichtquelle, ein Computer-Datenverarbeitungssystem und eine Bildschirmausgabe aufweisen kann. Insbesondere kann hier beispielsweise ein VektorVision®-System der Firma BrainLAB zur Anwendung kommen.

In der Figur 2 ist noch genauer zu sehen, dass die Schneidführung 2 einen Führungsschlitz 22 für ein chirurgisches Werkzeug aufweist, beispielsweise eine Säge. Die Ortungsreferenz 6 ist drehbar an der Schneidführung 2 angebracht, und zwar damit sie so ausgerichtet werden kann, dass sie nicht im Weg ist und vom Navigationssystem (Kamera-Trackingeinheit) gut sichtbar ist. Wegen ihrer Drehbarkeit kann sie nicht die genaue Lage der Justierungseinrichtung 4 an das Navigationssystem übermitteln, jedoch kann sie die Lage der Schnittebene (Schneidführung) übermitteln, welche durch den Schlitz 22 gebildet wird.

Um eine erfindungsgemäße Benutzerführung bereitstellen zu können, ist es notwendig, die räumliche Lage der Justierungseinrichtung 4 (vor allem in Bezug auf den zu bearbeitenden Knochen) zu kennen. Hierzu dient in einer ersten Ausführungsform der Registrierungspunkt 5, der an dem oberen Ende der Justierungseinrichtung 4 als Vertiefung ausgeführt ist. In eine solche Vertiefung kann beispielsweise ein navigierbarer Zeigestab mit der Spitze eingebracht werden, um ihre räumliche Lage mit dem Navigationssystem festzustellen. Die dadurch erhaltenen Informationen kombiniert mit den Informationen über die Lage der Ortungsreferenz sind ausreichend, um die Lage der Justierungsvorrichtung am Knochen zu bestimmen und anschließend die Anweisungen für den Einstellvorgang zum Erreichen der Soll-Schnittebene abzuleiten.

Die Justierungseinrichtung 4 hat drei Handräder 42, 43 und 44, mit denen die Schneidführung 2 um zwei nichtparallele Achsen gekippt und in der Höhe verstellt werden kann. Damit können in dem durch den Mechanismus begrenzten Verstellbereich der Justierungseinrichtung grundsätzlich alle Neigungsstellungen für die Schnittebene erreicht werden.

Um eine Justierung des Knochenschneidblockes durchzuführen, muss der Benutzer nun folgende Arbeitsschritte erledigen:

Zunächst wird das Bein im Navigationssystem registriert, dies geschieht mittels der Referenzen 11 und 21 für die Knochen 10 und 20 (Figur 1), die charakteristischen Achsen und Ebenen des Beines können damit vom Navigationssystem errechnet werden. Ebenfalls vorab sind die Handräder 42, 43 und 44 der Justierungseinrichtung 4 auf ihre Nullwerte einzustellen, um einen maximalen Einstellungsbereich in allen Richtungen und Freiheitsgraden zur Verfügung zu stellen.

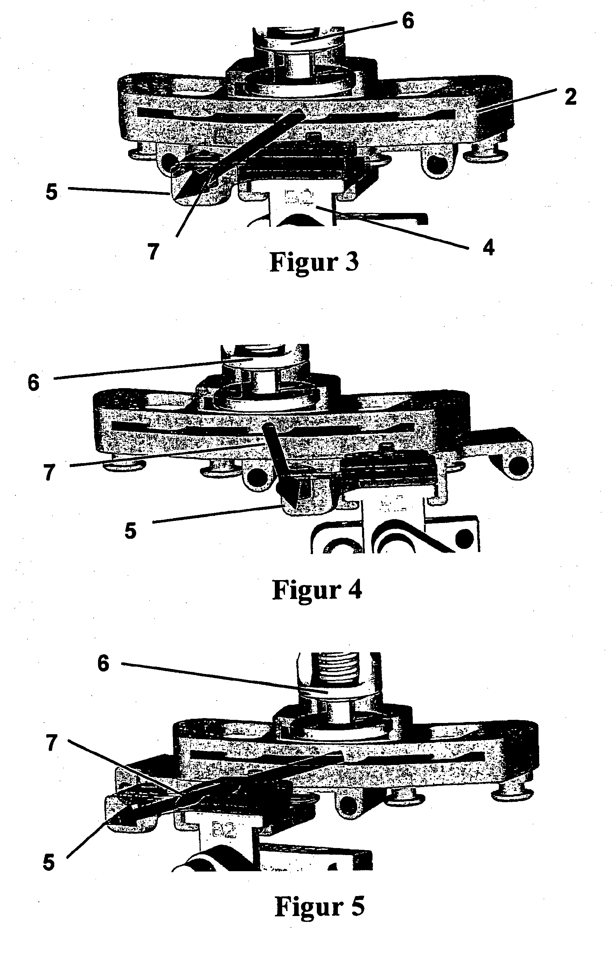

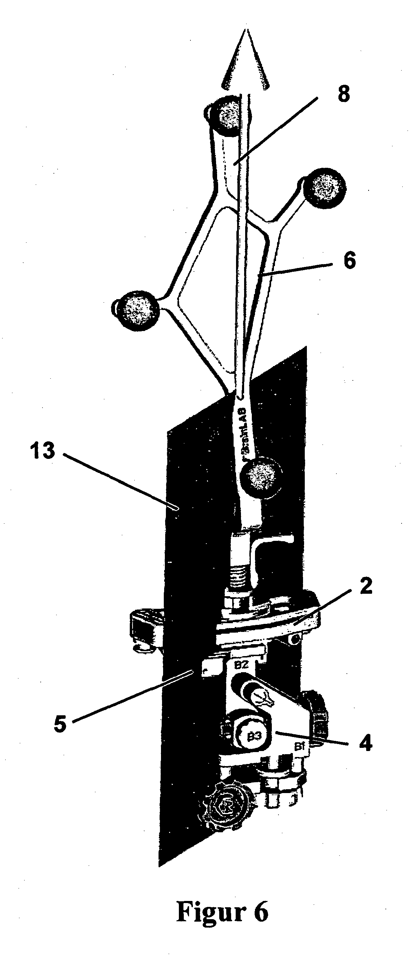

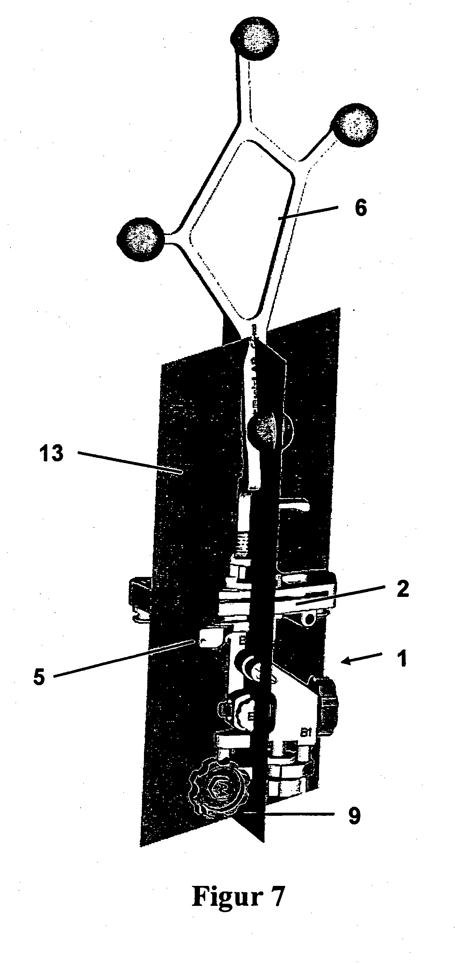

Wenn mehrere Konfigurationen für die Stellung der Schneidführung 2 gegenüber der Justierungseinrichtung 4 möglich sind, wird die Schneidführung 2 nunmehr in der gewünschten Stellung an der Justierungseinrichtung 4 montiert. Zur Erläuterung wird hier auf die Figuren 3 bis 5 Bezug genommen, die jeweils unterschiedliche Stellungen der Schneidführung 2 gegenüber der Justierungseinrichtung 4 zeigen. Die Pfeile 7 deuten hier immer auf unterschiedliche Abstände (als Werte im Navigationssystem hinterlegt) zwischen einem gewählten aber fixen Punkt an der Schneidführung 2, welcher idealerweise durch die Drehachse der Ortungsreferenz verläuft, und dem Registrierungspunkt 5. Wenn also solche unterschiedlichen Konfigurationen möglich sind (wie z.B. gemäß den Figuren 3 bis 5), muss dem unterstützenden Navigationssystem mitgeteilt werden, in welcher Beziehung die Ortungsreferenz 6 zur Justierungseinrichtung 4 liegt, und dies geschieht durch die Ermittlung des Abstandes zwischen der Achse der drehbaren Ortungsreferenz 6 und dem Registrierungspunkt 5. Weil die Ortungsreferenz 6 drehbar ist, ist der zusätzliche Registrierungspunkt 5 notwendig, um die Registrierungsebene 13 aufzubauen, welche für die unterschiedlichen Konfigurationen (Figur 3 bis 5) unterschiedliche Winkel zur Symmetrieebene des Schneidblocks hat. In der Figur 6 ist die Registrierungsebene aufgezeigt und mit dem Bezugszeichen 13 versehen worden; der Pfeil mit dem Bezugszeichen 8 bezeichnet die Drehachse der Ortungsreferenz 6. Die Figur 7 zeigt dann den Unterschied zwischen der Registrierungsebene 13 und der Symmetrieebene des Knochenschneidblockes 1, wobei die Symmetrieebene in Figur 7 mit dem Bezugszeichen 9 gekennzeichnet wurde.

Die bestimmte Pfeillänge für den Pfeil 7 in den Figuren 3 bis 5 wird dann verwendet, um den Koordinationswinkel zwischen der Registrierungsebene und der Symmetrieebene des Knochenschneidblocks zu finden, welche im Datensatz des Navigationssystems hinterlegt ist.

Wenn Schneidführung 2 und Justierungseinrichtung 4 in gewünschter Konfiguration miteinander verbunden sind wird die Ortungsreferenz 6 an der Schneidführung 2 angebracht; und der Knochenschneidblock 1 kann im zusammengesetzten Zustand an jedweder Stelle nahe des Resektionsbereiches am Knochen angesetzt (angeschraubt) werden.

Hierauf erfolgt die Registrierung des Knochenschneidblocks bzw. der Justierungseinrichtung 4 (und der in dieser Ausführungsform damit fest verbundenen Schneidführung 2) durch das Registrieren des Registrierungspunktes 5, indem dieser mit einem navigierten Zeigestab angefahren wird, dessen Spitze in der Vertiefung zur Ruhe kommt.

Wenn verschiedene Konfigurationen, das heißt Relativlagen zwischen Schneidführung 2 und Justierungseinrichtung 4 möglich sind (Figuren 3 bis 5) wird nunmehr der Abstand zwischen der Achse der Ortungsreferenz 6 und dem Registrierungspunkt 5 errechnet und diese Abstände werden mit dem Winkel zwischen Registrierungsebene 13 und Symmetrieebene 9 des Schneidblocks korreliert, welche zuvor in der Datenbank abgespeichert worden sind.

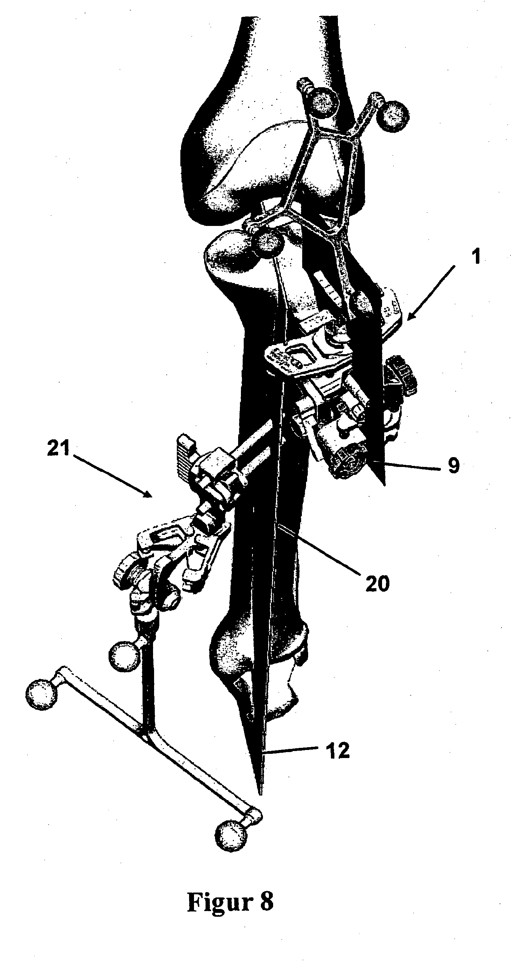

Mit den wie oben beschrieben berechneten Ebenen (Registrierungsebene, Symmetrieebene) kann per Software dann die Position des Schneidblocks (insbesondere der Justierungseinrichtung) an dem Knochen errechnet werden, wobei die errechnete Symmetrieebene mit der Sagittalebene des Knochens verglichen wird. Die Figur 8 stellt den Unterschied zwischen der Symmetrieebene des Schneidblocks und der Sagittalebene des zu behandelnden Knochens dar. Die Proportionen für die Einstellungen an den Handrädern 42, 43 und 44 der Justierungseinrichtung 4 können dann errechnet und für den Benutzer ausgegeben werden, beispielsweise auf dem Bildschirm des Navigationssystems. Dabei wird z.B. immer angegeben, welches Rad zu drehen wäre (z.B. durch Farbkodierung leicht unterscheidbar), bis ein Stopp-Signal erfolgt, und dies für jedes Rad, so dass die Soll-Schnittebene in nur drei Schritten eingestellt werden kann. Der Softwarealgorithmus berücksichtigt dabei die sich gegenseitig beeinflussenden Freiheitsgrade und berechnet genau die Werte für die Verstellung in den einzelnen Freiheitsgraden, die die Ist-Schnittebene in die Soll-Schnittebene überführen. Dabei wird beim ersten oder zweiten Einstellschritt nicht unbedingt einer der Schnittebenenparameter seinen Sollwert erhalten, was ein Unterdrücken der charakteristischen Werte während der Benutzerführung sinnvoll macht, um den Benutzer mit den mitunter vom Sollwert abweichenden Angaben nicht zu verwirren. Eine günstige Benutzerführung ist die Darstellung des zu betätigenden Einstellmittels (z.B. nach Farbe unterscheidbar) solange bis dessen Sollwert erreicht ist. Dieser Prozess wird solange wiederholt bis alle notwendigen Manipulationen der Justierungseinrichtung durchgeführt worden sind. Danach wird das Ergebnis als Vergleich zwischen Soll- und Ist-Schnittebene dargestellt.

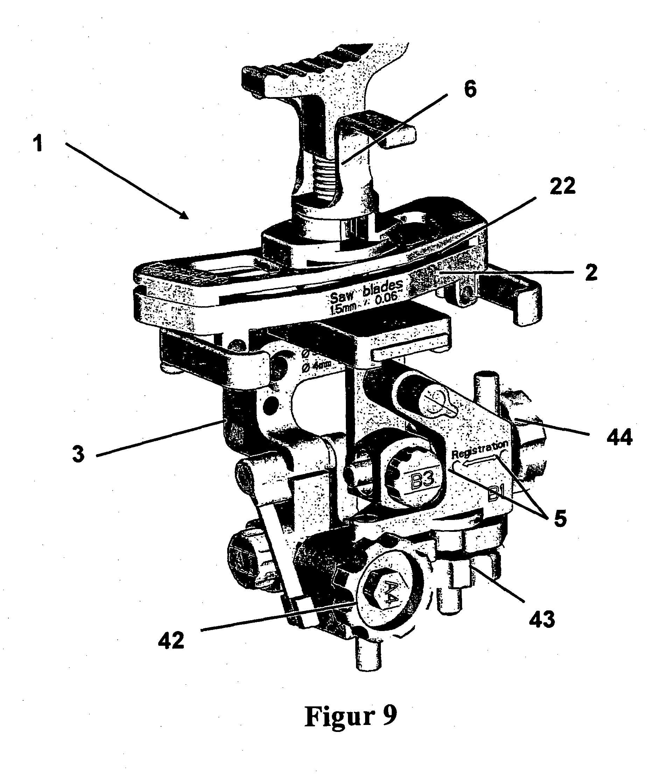

Figur 9 zeigt eine zweite Ausführungsform des erfindungsgemäßen Schneidblocks wobei hier ein zweidimensionales Registrierungselement 5 zur Anwendung kommt was mitunter dann sinnvoll ist, sobald keine vordefinierten Konfigurationen zwischen Justierungseinrichtung 4 und Schneidführung 2 vorhanden oder möglich sind und somit auch nicht über charakteristische Abstände 7 mit in der Datenbank hinterlegten Winkelbeziehungen korreliert werden können. Die Verwendung von zweidimensionalen Registrierungselementen erlaubt demnach die freie Bewegung der Schneidführung 2 innerhalb der Schnittebene, wobei die Informationen bezüglich der Schnittebene wie gewohnt über die Ortungsreferenz 6 an das Navigationssystem übergeben werden und zusätzlich mit Hilfe des zweidimensionalen Registrierungselementes (z.B. zwei Punkte oder eine Bohrung die den Normalvektor einer Ebene bilden) die Lage der Justierungsvorrichtung am Knochen bestimmt wird.

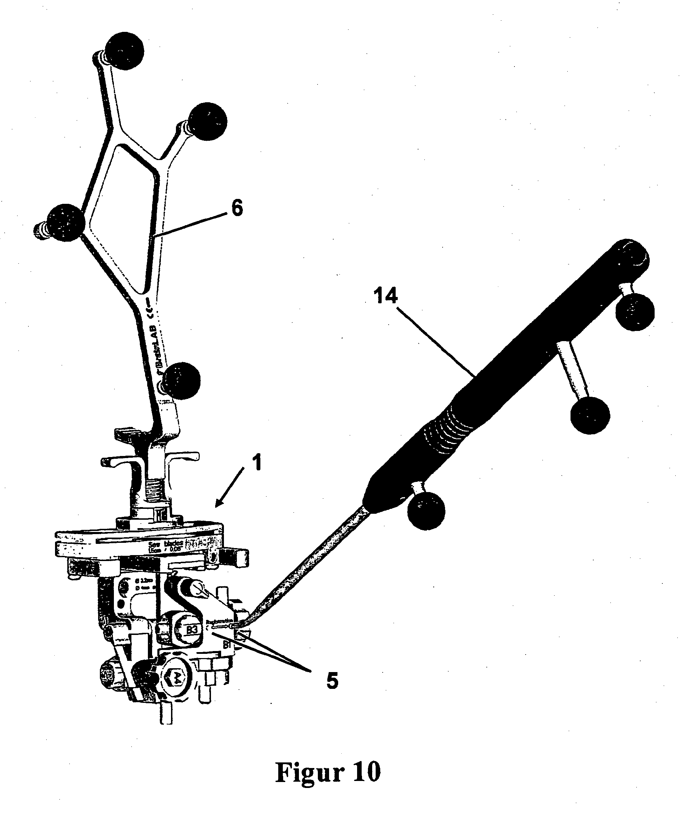

Figur 10 zeigt den Registrierungsprozess eines Schneidblocks mit einem in diesem Fall zweidimensionalen Registrierungselement 5, wobei mit einem vom Navigationssystem verfolgbaren Zeigestab 14 die Vertiefungen an der Justierungseinrichtung abgegriffen und zur Berechnung der Registrierungsebene mit einem ebenfalls erfindungsgemäßen Softwarealgorithmus verwendet werden.

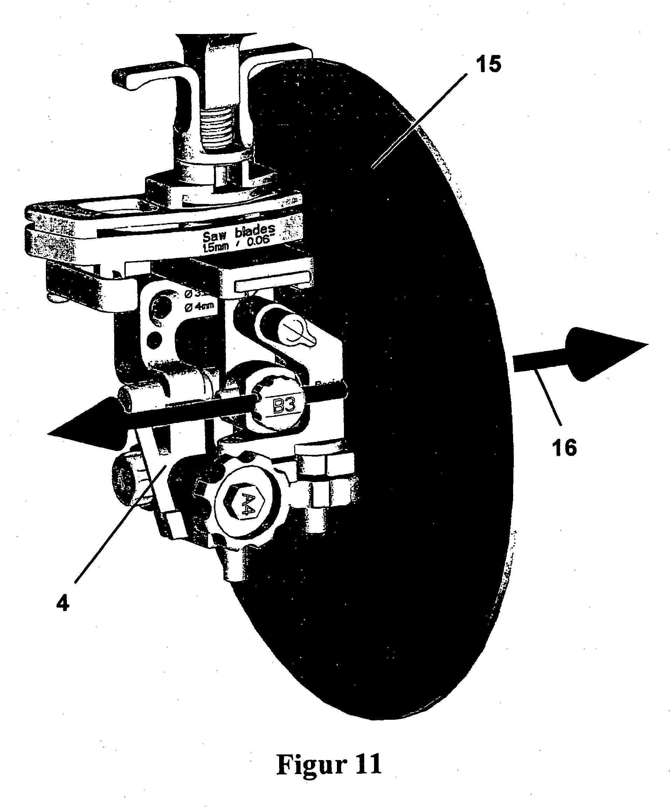

Figur 11 zeigt die Bildung der Registrierungsebene 15 am Beispiel eines Schneidblocks mit zweidimensionalem Registrierungselement, wobei die Verbindungslinie der Registrierungspunkte dem Normalenvektor 16 der Registrierungsebene entspricht der in der gezeigten Ausführungsform parallel zur Symmetrieebene der Justierungseinrichtung ist.

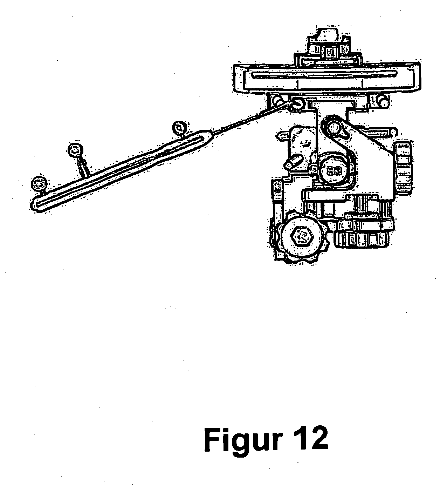

Figur 12 zeigt die Darstellung der Anweisung für den Benutzer bei der Durchführung des Registrierungsprozesses, bei dem die räumliche Lage der Justierungseinrichtung (hier mit eindimensionalem Registrierungselement) mit Hilfe eines navigierten Zeigstabs bestimmt wird.

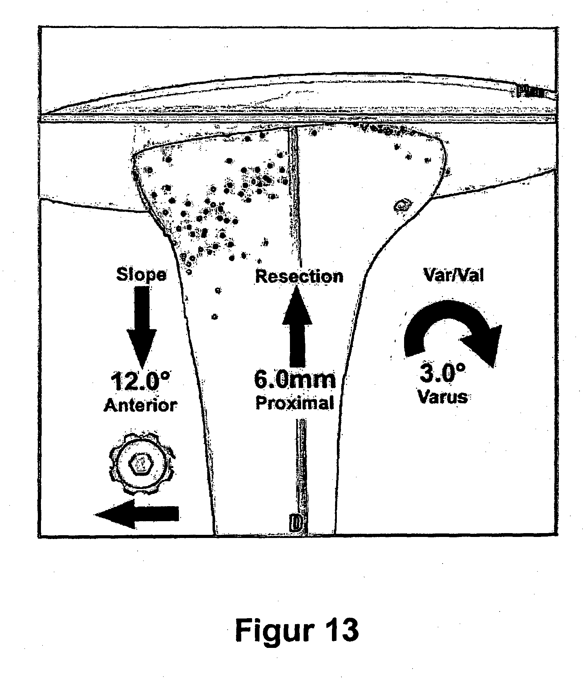

Figur 13 zeigt eine Möglichkeit der Darstellung für die Führung des Benutzers bei der Einstellung der Justierungseinrichtung in Bezug auf den Freiheitsgrad Slope der Schnittebene. Entsprechend der erforderlichen Drehbewegung ist dem zugehörigen Einstellmittel ein Richtungspfeil zugeordnet, wobei dieser solange angezeigt wird bis die Vorgaben des Navigationssystems vom Benutzer erfüllt worden sind.

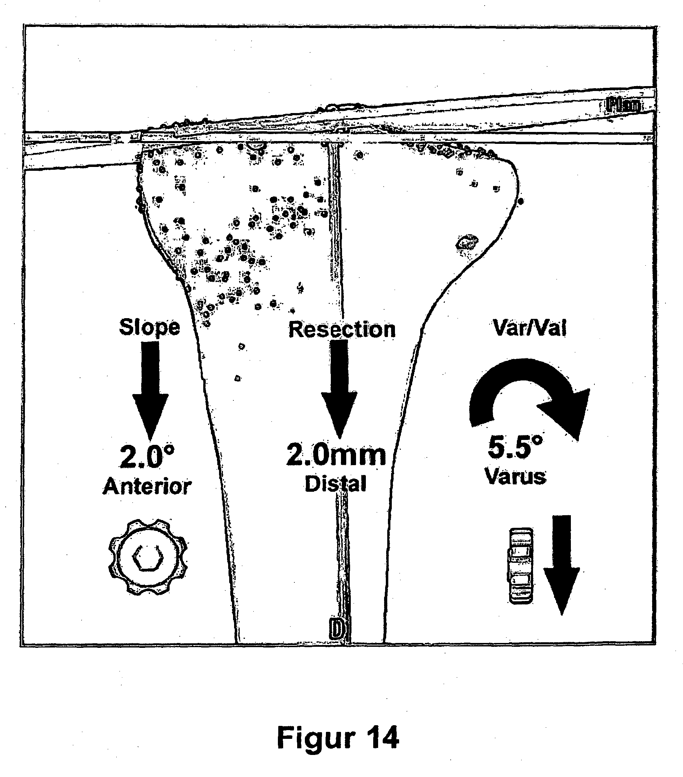

Figur 14 zeigt eine Möglichkeit der Darstellung für die Führung des Benutzers bei der Einstellung der Justierungseinrichtung in Bezug auf den Freiheitsgrad Varus/Valgus der Schnittebene. Entsprechend der erforderlichen Drehbewegung ist dem zugehörigen Einstellmittel ein Richtungspfeil zugeordnet, wobei dieser solange angezeigt wird bis die Vorgaben des Navigationssystems vom Benutzer erfüllt worden sind.

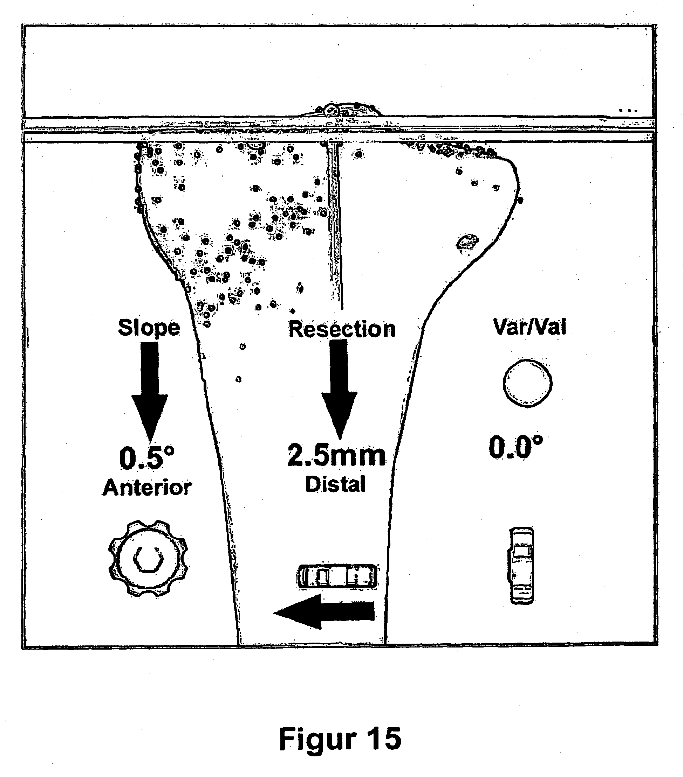

Figur 15 zeigt eine Möglichkeit der Darstellung für die Führung des Benutzers bei der Einstellung der Justierungseinrichtung in Bezug auf den Freiheitsgrad Resektionshöhe der Schnittebene. Entsprechend der erforderlichen Drehbewegung ist dem zugehörigen Einstellmittel ein Richtungspfeil zugeordnet, wobei dieser solange angezeigt wird bis die Vorgaben des Navigationssystems vom Benutzer erfüllt worden sind.

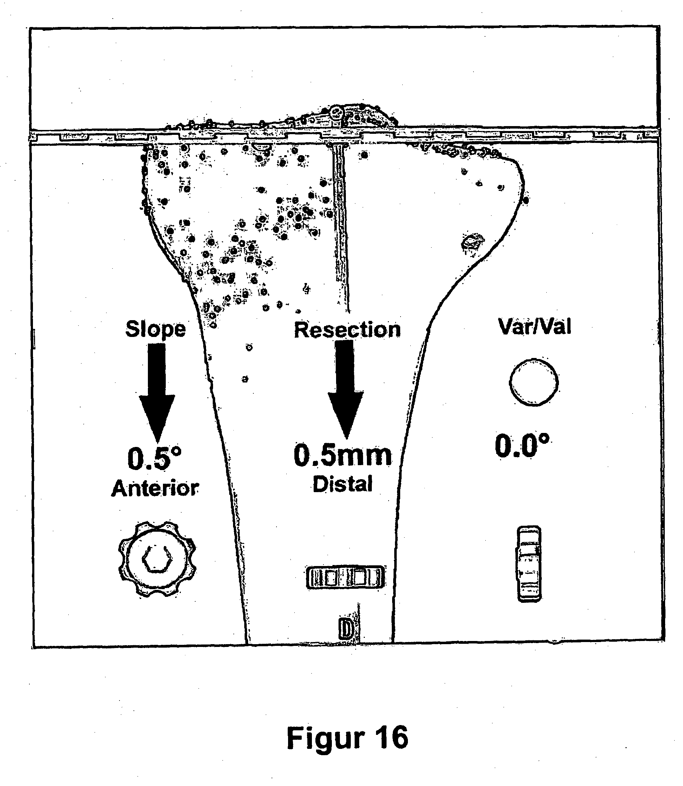

Figur 16 zeigt eine Möglichkeit der Darstellung der Informationen für den Benutzer nach Abschluss des Justierungsprozesses. Alle Einstellmittel werden ohne Anweisung zur weiteren Drehung angezeigt (keine Pfeile sichtbar) nachdem die Ist-Schnittebene der Soll-Schnittebene in vorgegebener Genauigkeit entspricht.

## Patentansprüche

1. Knochenschneidblock (1) mit einer Schneidführung (2), mit einer an der Schneidführung (2) versbellbar z.B. drehbar angebrachten Ortungsreferenz (6), die es gestattet, die Schnittebene der Schneidführung (2) räumlich zu bestimmen, mit einer Befestigung (3), die an einem Knochen befestigt werden kann, mit einer Justierungseinrichtung (4) zwischen Befestigung (3) und Schneidführung (2), wobei mittels der Justierungseinrichtung (4) die Schnittebene der Schneidführung (2) gegenüber dem Knochen eingestellt werden kann, und mit einem eine fest an der Justierungseinrichtung (4) angeordnete Referenz umfassenden Registrierungselement (5), mit dem die räumliche Lage der Justierungseinrichtung (4) bestimmbar ist, wobei die Referenz punktförmig ist oder eine zweidimensionale Ausdehnung aufweist.

2. Knochenschneidblock nach Anspruch 1, **dadurch gekennzeichnet, dass** die Justierungseinrichtung (4) Einstellmittel aufweist, mittels denen die Schneidführung (2) um zwei nichtparallele Achsen gedreht und in der Höhe verstellt werden kann, insbesondere jeweils ein Einstellmittel für jeden Freihertsgrad, speziell drei Handrad-Schraubeinstellvorrichtungen (42, 43, 44).

3. Knochenschneidblock nach Anspruch 1 oder 2, **dadurch gekennzeichnet, dass** das Registrierungselement (5) der Justierungseinrichtung (4) an einem für den einzelnen Knochenschneidblock (1) charakteristischen Ort angeordnet ist.

4. Knochenschneidblock nach einem der Ansprüche 1 oder 2, **dadurch gekennzeichnet, dass** das Registriecungselement (5) der Justierungseinrichtung (4) in einem vorgegebenen Abstand von der Ortungsreferenz (6) angeordnet ist.

5. Knochenschneidblock nach einem der Ansprüche 1 oder 2, **dadurch gekennzeichnet, dass** die Schneidführung (2) inklusive der an ihr befestigten Ortungsreferenz (6) für verschiedene Konfigurationen in einem definiert voreinstellbaren Abstand vom Registrierungselement (5) der Justierungseinrichtung (4) angeordnet ist.

6. Knochenschneidblock nach einem der Ansprüche 1 bis 4, **dadurch gekennzeichnet, dass** das Registrierungselement (5) eine Vertiefung an einer Stelle der Oberfläche der Justierungseinrichtung (4) ist.

7. Knochenschneidblock nach einem der Ansprüche 1 bis 4, **dadurch gekennzeichnet, dass** das Registrierungselement (5) eine zweidimensionale Ausdehnung hat, insbesondere eine Bohrung an einer Stelle der Justierungseinrichtung (4) ist oder durch zwei Vertiefungen an der Oberfläche der Justierungseinrichtung (4) gebildet wird, wobei die räumliche Lage beider Elemente mit einem navigierten Zeigestab ermittelt werden kann.

8. Knochenschneidblock nach Anspruch 1 oder 2, **dadurch gekennzeichnet, dass** die Justierungsvorrichtung (4) eine Stellvorrichtung für die Winkel-Relativstellung zwischen der Ortungsreferenz (6) und der Justierungseinrichtung (4) umfasst, die lediglich vorbestimmte und definierte Relativstellungen zulässt und einrastet und die eingestellte Relativstellung anzeigt oder vom Navigationssystem ausgelesen werden kann.

9. Knochenschneidblock nach Anspruch 2 wobei die sich farblich unterscheidenden Einstellmittel auch durch farbliche Kenntlichmachung in der Bildschirmanzeige leichter für den Benutzer zu identifizieren sind.

## Claims

1. A bone incision block (1), comprising: an incision guide (2) to which a localisation reference (6) is adjustably, for example rotatably, attached which allows the incision plane of the incision guide (2) to be spatially determined; a fastening (3) which can be fastened to a bone; an adjustment means (4) between the fastening (3) and the incision guide (2), wherein the incision plane of the incision guide (2) can be set relative to the bone by means of the adjustment means (4); and a registration element (5) which includes a reference which is arranged fixed to the adjustment means (4), wherein the spatial position of the adjustment means (4) can be determined using the registration element (5), and wherein the reference is puncticular or has a two-dimensional extent.

2. The bone incision block according to claim 1, **characterised in that** the adjustment means (4) comprises setting media, by means of which the incision guide (2) can be rotated around two non-parallel axes and adjusted in height, in particular one setting medium for each degree of freedom respectively, specifically three hand wheel screw setting devices (42, 43, 44).

3. The bone incision block according to claim 1 or 2, **characterised in that** the registration element (5) of the adjustment means (4) is arranged at a location characteristic of the individual bone incision block (1).

4. The bone incision block according to any one of claims 1 or 2, **characterised in that** the registration element (5) of the adjustment means (4) is arranged at a preset distance from the localisation reference (6).

5. The bone incision block according to any one of claims 1 or 2, **characterised in that** the incision guide (2), including the localisation reference (6) fastened to it, is arranged for various configurations at a distance, which can be pre-set in a defined way, from the registration element (5) of the adjustment means (4).

6. The bone incision block according to any one of claims 1 to 4, **characterised in that** the registration element (5) is a depression at a point on the surface of the adjustment means (4).

7. The bone incision block according to any one of claims 1 to 4, **characterised in that** the registration element (5) has a two-dimensional extent and is in particular a bore at a point on the adjustment means (4) or is formed by two depressions on the surface of the adjustment means (4), wherein the spatial position of the two elements can be ascertained using a navigated pointer.

8. The bone incision block according to claim 1 or 2, **characterised in that** the registration device (4) includes a setting device for the relative angular position between the localisation reference (6) and the adjustment means (4) which only allows and locks in predetermined and defined relative positions, and **in that** the relative position set can be indicated or can be read from the navigation system.

9. The bone incision block according to claim 2, wherein the setting media which differ in colour are also to be more easily identified by the user in the screen display by coloured marking.

## Revendications

1. Corps de coupe d'un os (1), comportant un guide de coupe (2), comportant une référence de repérage (6), qui est montée réglable et rotative sur le guide de coupe (2) et permet de déterminer dans l'espace le plan de coupe du guide de coupe (2), comportant une fixation (3) qui peut être fixée à un os, comportant un dispositif d'ajustage (4) entre la fixation (3) et le guide de coupe (2), ledit dispositif d'ajustage (4) permettant de régler le plan de coupe du guide de coupe (2) par rapport à l'os, et comportant un élément enregistreur (5) qui comprend une référence agencée sur le dispositif d'ajustage (4) et permet de déterminer la position dans l'espace du dispositif d'ajustage (4), ladite référence étant en forme de point ou présentant une étendue bidimensionnelle.

2. Corps de coupe d'un os selon la revendication 1, **caractérisé en ce que** le dispositif d'ajustage (4) comporte des moyens de réglage qui permettent de faire tourner le guide de coupe (2) autour de deux axes non parallèles et de le régler en hauteur, en particulier respectivement un moyen de réglage pour chaque degré de liberté, spécifiquement trois dispositifs de réglage à vis et molette (42, 43, 44).

3. Corps de coupe d'un os selon la revendication 1 ou 2, **caractérisé en ce que** l'élément enregistreur (5) du dispositif d'ajustage (4) est agencé en un lieu caractéristique pour le corps de coupe d'un os (1) individuel.

4. Corps de coupe d'un os selon la revendication 1 ou 2, **caractérisé en ce que** l'élément enregistreur (5) du dispositif d'ajustage (4) est agencé à une distance prédéfinie de la référence de repérage (6).

5. Corps de coupe d'un os selon la revendication 1 ou 2, **caractérisé en ce que** le guide de coupe (2), y compris la référence de repérage (6) fixée à ce dernier, est agencé pour différentes configurations à une distance définie préréglable de l'élément enregistreur (5) du dispositif d'ajustage (4).

6. Corps de coupe d'un os selon l'une des revendications 1 à 4, **caractérisé en ce que** l'élément enregistreur (5) est un creux à un emplacement de la surface du dispositif d'ajustage (4).

7. Corps de coupe d'un os selon l'une des revendications 1 à 4, **caractérisé en ce que** l'élément enregistreur (5) a une étendue bidimensionnelle, en particulier est une forure à un emplacement du dispositif d'ajustage (4) ou est formé par deux creux dans la surface du dispositif d'ajustage (4), la position dans l'espace des deux éléments pouvant être calculée au moyen d'une tige indicatrice pilotée.

8. Corps de coupe d'un os selon la revendication 1 ou 2, **caractérisé en ce que** le dispositif d'ajustage (4) comporte un dispositif de réglage pour la position angulaire relative entre la référence de repérage (6) et le dispositif d'ajustage (4), qui autorise uniquement des positions relatives prédéterminées et définies et s'enclenche et la position relative réglée peut être indiquée ou peut être lue par le système de navigation.

9. Corps de coupe d'un os selon la revendication 2, dans lequel des moyens de réglage, distincts par leur couleur, sont aussi plus faciles à identifier sur l'écran par l'utilisateur grâce à la reconnaissance de leur couleur.
